# EUROPEAN PATENT APPLICATION

(11) **EP 2 351 771 A1**
(43) Date of publication of application: **03.08.2011**
(21) Application number: 08877453.4
(22) Date of filing: 01.11.2008
(51) Int. Cl.: C07K 14/15, A61K 39/00, A61K 38/16, A61P 37/00

(54) **IMMUNOSUPPRESSIVE PEPTIDE**

(30) Priority: 15.10.2008 RU 2008140688
(71) Applicant: Shurdov, Mihail Arkadievich, Moscow 124305 (RU)
(72) Inventor: BLINOV, Vladimir Mihailovich, Novosibirskaya obl. 630559 (RU)
(74) Representative: Gerasimovic, Liudmila
(86) International application number: PCT/RU2008/000682
(87) International publication number: WO 2010/044691

(57) **Abstract**

This invention relates to medicine, namely to the family of immunosuppressive peptides (ISP) suppressing a wide range of specific links (components) of human immune system. The desired result of making it possible to extend functionalities is achieved by means of an immunosuppressive peptide, the formula of which has variable positions with two (X) or three (Y) amino-acid substitutions : 
D-loop C-loop N-loop

| | | * disulfide bond * | |

| | | | | | |

X1-L-D-X2-L-T-A-X3-Y1-G-G-Y2-C-Y3-X4-L-Y4-E-X5-C-C-X6-X7-Y5-N-Q-S-G-Y6-V

|

free
 X1={G,A}
X2={L,M}
X3={E,A}
X4={F,A}
X5={F,K}
X6={F,Y}
X7={Y,W}
Y1={K,R,Q}
Y2={L,I,T}
Y3={L,I,T}
Y4={G,D,N}
Y5={V,L,T}
Y6={I,L,K}.

For C and D type exogenous retroviruses (Human T-cell leukemia virus type I/II, imian Mason-Pfizer virus, Simian retrovirus SRV-1/2; Squirrel monkey retrovirus) the formula is: 
D-loop C-loop N-loop
* disulfide bond *
G-L-D-L-L-X1-X2-E-Q-G-G-X3-C-X4-A-X5-Q-E-X6-C-C-F-X7-X8-N-X9-S-X10-X11-X12
 X1={F,T}
X2={W,A}
X3={L,I}
X4={L,K}
X5={L,I}
X6={Q,K}
X7={L,Y}
X8={A,-}
X9={I,K}
X10={N,G}
X11={I,T,S}
X12={V,H}.
, and peptide library is 
G-I--D-L-- L-X1-X2-E--Q--G-G-X3-C-X4-A--X5-Q--E-X6-C-C-F--X7-X8- N-X9-S-X10-X11-X12
X1-L-D-X2-L-T--A--X3-Y1-G-G-Y2-C-Y3-X4-L---Y4-E-X5-C-C-X6-X7-Y5-N-Q--S-G---Y6--V
### # ### # # ### # #,
 where # - designates coinciding ammo-acid residues.

## Description

This invention relates to medicine, namely to the family of immunosuppressive peptides (ISP) suppressing a wide range of specific links (components) of human immune system that allows to use this peptide family (as well as antibodies to them) in diagnostics of body organs and tissues sensitive to certain immunosuppressive peptide classes, and in transplantation of body organs and tissues during surgeries.

Compositions for vaccine creation in order to prevent and cure virus diseases are known. This therapy includes antigenic peptide families in which antigenic peptides differ from each other by the location of at least one amino acid [RU 2312941, A 61 K 39/00, 20.12.2007]. The drawback of such compositions is relatively limited functional capabilities since they do not possess pronounced immunosuppressive features.

A peptide possessing immunosuppressive features with H-A-D-Trp-J formula, where A-D-Glu or D-iGlu, J-OH or a substituted amide (C₁-C₃) [RU 2123859, A 61 K 38/04, 27.12.1998] is the closest to the proposed peptide.
Relatively limited functional capabilities comprise a drawback of this similar solution since they possess pronounced immunosuppressive features of a limited class of specific immune system components only. The required result implies expansion of the functional capabilities.

The desired result may be achieved by immunosuppressive peptide having formula with variable positions and two (X) or three (Y) amino-acid replacements: 
D-loop C-loop N-loop

| | | * disulfide bond * | |

| | | | | | |

X1-L-D-X2-L-T-A-X3-Y1-G-G-Y2-C-Y3-X4-L-Y4-E-X5-C-C-X6-X7-Y5-N-Q-S-G-Y6-V

|

free
 X1-{G,A}
X2-{L,M}
X3-{E,A}
X4-{F,A}
X5-{E,K}
X6={F,Y}
X7={Y,W}
Y1={K,R,Q}
Y2-{L,I,T}
Y3-{L,I,T}
Y4-{G,D,N}
Y5={V,L,T}
Y6-{I,L,K}

Moreover, the desired result may be achieved by the way that for C and D type exogenous retroviruses (Human T-cell leukemia virus type I/II, Simian Mason-Pfizer virus, Simian retrovirus SRV-1/2, Squirrel monkey retrovirus) the formula is: 
D-loop C-loop N-loop

* disulfide bond *

G-L-D-L-L-X1-X2-E-Q-G-G-X3-C-X4-A-X5-Q-E-X6-C-C-F-X7-X8-N-X9-S-X10-X11-X12
 X1={F,T}
X2={W,A}
X3={L,I}
X4={L,K}
X5={L,I}
X6={Q,K}
X7={L,Y}
X8={A,-}
X9={I,K}
X10={N,G}
X11={I,T,S}
X12-{V,H}.

Moreover, the desired result may be achieved by the way of peptide library: 
G-L-D-L-- L-X1-X2-E--Q--G-G-X3-C-X4-A--X5-Q--E-X6-C-C-F--X7-X8- N-X9-S-X10-X11-X12
X1-L-D-X2-L-T--A--X3-Y1-G-G-Y2-C-Y3-X4-L--Y4-E-X5-C-C-X6-X7-Y5-N-Q--S-G---Y6--V
### # ### # # ### # #,
 where # - designates coinciding amino-acid residues.

The immunosuppressive peptide suggested may be obtained and used in the following way.

Theoretical substantiation of the suggested solution should be carried out preliminarily. The invention refers to the new immunosuppressive peptide families suppressing a specific component of immune system.
In human genome, the elements of endogenous human retroviruses (HERV) are embedded into 17317 genes from all 26981 genes known at present. 898 expressing genes are under control of HERV elements (LTR-promoting agents). Terminal repeats (LTR) of endogenous human retroviruses comprise 8% of the whole length of human genome. Expression of LTR-promoting agents increases in placenta and some tumourous cells.

Endogenous human retroviruses include the following genes: GAG- core proteins; PRO-proteases; RT- reverse transcriptases; RNase H - nucleases; IN- intcgrases; ENV- coat proteins.
Besides full LTR-HERV-LTR endogenous human retroviruses and their damaged copies, there are individual copies of GAG, PRO, RT, RNaseH, IN and ENV genes in human genome. Coat proteins (ENV) of endogenous retroviruses in which the so called immunosuppressive domains (ISD) arc coded, are of special interest.

We have mapped about 1000 immunosuppressive domains in 24 human chromosomes. Regardless of the fact whether an immunosuppressive domain is included into an endogenous retrovirus or not, we have revealed a conservative domain with the length of 90 base pairs which represents an evolutionary fixed exon encoding an immunosuppressive peptide being 30 amino acid residues in size.

The evolution of immunosuppressive exons in human genome is connected not only with endogenous retroviruses, but represents also an individual evolution branch of evolutionary fixed immunosuppressive exons (ISE) movement in different human chromosomes.
The major part of immunosuppressive exons (ISE) is mapped in intercistron areas of human genome and a small part of immunosuppressive exons only is mapped in introns of other genes. Exactly these copies of immunosuppressive exons may be included into expressing genes by means of alternative splicing.
The activation of immunosuppressive exons which are localized in intergenic sequences is likely to be performed by means of promoting agents for LTR/SINE/LINE genetic elements located in proximity of immunosuppressive exons. About 30% of all immunosuppressive exons represent damaged (relict) forms of exons in which exon-intron borders are damaged or early termination of translation or deletions/insertions are observed resulting in translation frames failure in immunosuppressive exons.

The formula for immunosuppressive peptides with indication of variable positions with two (X) or three (Y) amino-acid replacements: 
D-loop C-loop N-loop

* disulfide bond *

X1-L-D-X2-L-T-A-X3-Y1-G-G-Y2-C-Y3-X4-L-Y4-E-X5-C-C-X6-X7-Y5-N-Q-S-G-Y6-V

free
 X1={G,A}
X2={L,M}
X3={E,A}
X4={F,A}
X5={E,K}
X6={F,Y}
X7={Y,W}
Y1={K,R,Q}
Y2={L,I,T}
Y3={L,I,T}
Y4={G,D,N}
Y5-{V,L,T}
Y6={I,L,K}.

For C and D type exogenous retroviruses (Human T-cell leukemia virus type I/II, imian Mason-Pfizer virus, Simian retrovirus SRV-1/2; Squirrel monkey retrovirus) the formula is: 
D-loop C-loop N-loop

| | | * disulfide bond * | |

| | | | | | |

G-L-D-L-L-X1-X2-E-Q-G-G-X3-C-X4-A-X5-Q-E-X6-C-C-F-X7-X8-N-X9-S-X10-X11-X12
 X1={F,T}
X2-{W,A}
X3={L,I}
X4-{L,K}
X5-{L,I}
X6={Q,K}
X7={L,Y}
X8={A,-}
X9={I,K}
X10={N,G}
X11={I,T,S}
X12={V,H}.

Comparison of two formulae for endogenous and exogenous retroviruses may be written as follows: 
G-L-D-L-- L-X1-X2-E--Q--G-G-X3-C-X4-A--X5-Q--E-X6-C-C-F--X7-X8- N-X9-S-X10-X11-X12
X1-L-D-X2-L-T--A--X3-Y1-G-G-Y2-C-Y3-X4-L--Y4-E-X5-C-C-X6-X7-Y5-N-Q--S-G---Y6--V
### # ### # # ### # #,
 where # - designates coinciding amino-acid residues

11 amino-acid residues coincide between them: L, D, L, G, G, C, E, C, C, N, S which provide the structural similarity of D-C-N loops.

Similar formulae are present for endogenous and exogenous retroviruses of mice, pigs, cats and birds for which the structural features of immunosuppressors remain the same as for humans, except for N-loops which are absent in pigs, mice and cats, while such N-loops remain in birds as in humans.

Thus, interspecific differences of immunosuppressors are connected first of all with the structures of N-loops, therefore interspecific rejection (incompatibility) of organs in different organisms is directly connected with high variability of COOH terminal N-loops of immunosuppressors.

D-loop consisting of six amino-acid residues (L-D-X2-L-T-A) is an analog of retroviral hexapeptide (LDLLFL) which possesses an immunosuppressing effect. Non-specific immunosuppressors (IEW, IDW, Deygin) also belong to this type of supressors (a negatively charged amino-acid among hydrophobic residues). A family of immunosuppressors-cyclosporines A (CyA), cyclic peptides (GLVLAALLV) obtained from fingi which contain hydrophobic amino-acid residues and are bound with phospholipids in membranes of sensitive cells. Affinity with membrane and presence of a charged amino acid (D, E) among hydrophobic amino acids is predetermined in the recognition of immunosuppressors' D-loop by specific receptors in cells sensitive to them.

Variable positions in D-loop give a large variety for immunosuppressive peptides and ways for immunosuppressive peptides to be bound with different receptors in sensitive cells and thus predetermine a tissue host range of immunosuppressors.

C-loop is a conformational loop including 12 amino-acid residues (G-G-Y2-C-Y3-X4-L-Y4-F-X5-C-C) which provides for the conformation of an immunosuppressive peptide due to the formation of disulfide bond between cysteines in positions 4 and 12. Free cysteine residue in position 11 (reactogenic) may either form dimeric bonds between two immunosuppressors, or a bond with tissue (organ) receptor for its class of immunosuppressors.

N-loop -in COOH terminal part of an immunosuppressor a peptide is mapped (N-Q-S-G-Y6-V) which includes a glycosylation site (NXS/T, where X means any amino acid except for Pro). As a result of immunosuppressive peptide modification and due to covalent addition to Asn(N) amino-acid residue - polysaccharides, COOH-terminus of an immunosuppressor becomes more hydrophilic and therefore receives an additional affinity with an immunosuppressive receptor (ISR).

Production of immunosuppressive peptides is performed in the following way. At least two methods may be used in order to receive immunosuppressive peptides:
cloning of a base (nucleotide) sequence corresponding with a peptide amino-acid residue in an expression vector with subsequent expression of a structure in E.coli cells and peptide recovery in pure form;
chemical synthesis of a peptide in solid phase.
Let us consider both methods.
Cloning method in an expression vector. Production of polypeptide in E.coli cells.
There is a possibility to express cloned genes in different procariotic and eukaryotic host cells in practice. In order to produce a large quantity of eukaryotic peptides cloning and expression of a gene corresponding with a eukaryotic peptide in E.coli cells are more convenient/ In this case effective and controlled synthesis of recombinant peptides may be performed.

Hybrid genes may be constructed in such a way that foreign proteins determined by them shall be localized in E.coli cells cytoplasm, or in case leader sequence is embedded into coding sequence they will be secreted outside via cell membrane. As a rule, when recombinant polypeptides remain in a cell, they are accumulated in large quantities (up to 25% of the total cell protein) than when they are secreted by a cell (less than 1% of the total cell protein). However, many polypeptides expressed into the cytoplasm form partially soluble aggregates, and in this case special methods of their transfer into soluble state are necessary while operating with them.

Taking into account the empirical character of dissolution methods and protein recovery selected for each individual protein, below is proposed the general scheme of active soluble protein products recovery from non-soluble proteins produced in E.coli cells cytoplasm and expressed into cytoplasm as soluble products or secreted by a cell.

Vectors used for the expression.
High level of accumulation of a foreign product in host cells is frequently conditional on the initiation of transcription by a strong promoting agent and the presence of a large quantity of heterologous gene copies in each cell. However, it is extremely important for the expression of plasmid genes to be controlled since plasmids remain in host cells during many generations. This is essential since the synthesis of recombinant products shall be the metabolic load on cells; cells which have lost plasmids soon advance in their growth comparing with those cells which contain plasmids during reproduction. Strict control over expression allows to obtain bigger mass of cells first of all which do not express a heterologous gene with further appearance of a small number of generations possessing expression of such gene.

The expression is controlled better when using plasmids with temperature-controlled transcription system containing such promoting agents as P_{R} and P_{L} obtained from λ phage. Control over the work of such promoting agents is performed, for example, by a temperature sensitive product of λ-repressor gene *cI*₈₅₇ denaturing at over 37°C. The other peculiarity of such vector lies in the fact that it contains two points of replication start. As a result, one can increase an effective accumulation of a product due to the possibility to control the number of plasmid copies in a cell. At replication start from one of the points, a small number of plasmids copies is formed, at replication start from the other point located under control of P_{R}, λ-promoting agent - a large number of copies is formed. Plasmid replication is carried out from a replication start point with small number of copies at 34°C and less. Replication process starting from the other point is controlled by *cI*₈₅₇-repressor. Thus, larger biomass of cells may be grown and then cultivation temperature may be raised up to 38-42 °C. In this case denaturation of *cI*₈₅₇-repressor takes place, as well as the increase of plasmid copies number and inclusion of an imbedded foreign gene expression as a consequence.
While using such expression vectors, large quantities of recombinant proteins may be obtained. Usually, they comprise 20-25% of the total cell protein.

The technology described allows to obtain such proteins in different quantities - from the volume of a laboratory flask to the volume of a fermenter (100-200 L).

### Expression methods.

There are two general approaches to intracellular expression of the closed genes.
According to the first approach, the corresponding gene may be cloned in one reading frame with synthetic or bacterial coding sequences and expressed with the hybrid protein formation. In the other approach, direct expression of an embedded gene is implemented.
The need in expression of eukaryotic polypeptides as part of hybrid proteins arose when it was discovered that the level of eukaryotic proteins' expression in E.coli cells was limited due to the fact that they had been recognized as foreign by the cell and therefore had been destroyed, that was vividly evident in the case with polypeptides of a small size.

Hybrid products which are accumulated in cells in large quantities are synthesized within ligation of a eukaryotic gene with a bacterial gene. However, the necessity in method allowing to split a hybrid protein correctly arises when a eukaryotic polypeptide is needed to be received in pure form. On the other hand, direct expression of one eukaryotic gene gives the possibility to receive the right protein product, However, primary translation products carry methionine residue on their N-terminus. There are enzymes in E.coli cells which perform efficient separation of methionine from natural proteins, if necessary, but in case of recombinant proteins such enzymes do not operate efficiently. Thus, proteins received as a result of direct expression of a eukaryotic gene may contain N-terminal methionine unusual for natural protein.

Proteins which include a signal sequence are able to be secreted via cytoplasmic membrane of E.coli into periplasmic space or in the direction of outer cell membrane and further into extracellular environment. Foreign proteins' expression with their further secretion has some advantages over expression of proteins remaining inside cells. If signal sequence is processed correctly, end amino acid of a recombinant protein will be identical to a natural protein. Secretion into periplasm may also prevent polypeptide degradation. The most significant advantage of expression systems of such type is the fact that disulphide bonds are formed in secretion process and active products having the right conformation are created.

### Insoluble proteins.

Insoluble recombinant proteins are usually accumulated in E.coli cells in the form of discrete incorporations (inclusions). In a light microscope, they look like big aggregates refracting the light frequently accumulated on cellular poles. Inclusion bodies are released from cells in destruction of cell walls and membrane remaining as usual in non-soluble form. Inclusion bodies posses a high level of density and they can be isolated by centrifugation at acceleration being from 500 to 12000 g. After this stage inclusion bodies (and not hybrid protein) are washed with solution solubilizing impurities. Different reagents are used in such washing. In this case it is important to use such concentration which ensures the minimal degree of dissolution.

E.coli cell wall has a complex structure and consists of three layers clearly distinguishable: outer, lipopolysaccharide layer to which a bilayer membrane (outer cell membrane) is adjacent, and peptidoglycane layer closely connected with the outer cell membrane. Cytoplasmic membrane is separated from the peptidoglycane layer by the periplasmic space.

Both E.coli cell wall and cytoplasmic membrane should be destroyed in order to release non-soluble or soluble proteins located in cytoplasm. E.coli cells may be lysed by such enzymes as lysozyme in combination with detergents. Hydrodynamic method (French press) or Manton-Gaulin homogenizer may be used. Destruction by ultrasound may also be used, but this method is applied in small volumes of solutions only.

As stated above, hybrid proteins consist of bacterial or synthetic polypeptide connected with a eukaryotic polypeptide. Usually it is necessary to separate a eukaryotic protein only.
The following approach is used in this case.

A hybrid gene, in the product of which there is a splitting point located between a chain section which is coded by bacterial or artificially synthesized sequence and a section which is coded by eukaryotic gene sequence, is being constructed.
There is a large number of approaches solving the problem of splitting which may be divided into, two main types in general: chemical and enzymatic.

The choice of a specific approach is determined by the features of a foreign protein, and ideal situation will be the possibility to use the restriction site absent in sequence of a foreign protein or peptide.

Below the variants of hybrid proteins' splitting are outlined.

| Recognizable sequence | Splitting agent |
|---|---|
| -Asp-Pro- | Acid pH |
| -Met- | Cyanogen bromide |
| -Arg- or - Lys- | Tripsin |
| -Arg- | Clostridial peptidase |
| -Lys- | Endoproteinase Lys-C |
| -(Asp)₄ - Lys- | Enterokinase |
| -Ile-Glu-Gly-Arg-x- | Xa Factor |
| -Pro-x-Gly-Pro-y- | Collagenase |

Hybrid proteins may be constructed in such a way that synthetic or bacterial sequences, connected to the desired gene, code polypeptides which are selectively joined to reagents used for chromatography.

The following combinations of hybrid protein-ligand are known:

| Hybrid protein | Ligand |
|---|---|
| β- galactosidase -X | n- aminophenyl -β-D-thiogalactoside |
| CAT-X | Chloramphenicol |
| Protein A-X | IgG |
| X-polyarginine | Cation exchanger |

In order to obtain the desired polypeptide in the right conformation a hybrid protein is primarily isolated as inclusion bodies, then splitting is performed along with the production of the desired polypeptide in soluble form, and renaturation of the isolated target product is carried out after all.

A range of reagents is used for the transfer of polypeptides into solution being a part of non-soluble inclusions. They include: guanidine chloride (5-8M), urea (6-8M), SDS, alkaline pH, acetonitrile /propanol.
After solubilization of the desired polypeptide its renaturation in buffer is performed. This buffer does not contain denaturants. This is achieved by means of either dialysis, or dilution of the isolated and solubilized polypeptide.
In the process of polypeptide renaturation, one of the determining parameters is its concentration since it is necessary that the intramolecular interactions prevailed over the intermolecular interactions.

### Soluble proteins.

There are examples when non-native polypeptides synthesized in E.coli remain soluble and are secreted efficiently into periplasmic space. Expression levels are not high in this case and comprise less than 1% of the total protein of E.coli cells in comparison with the same in intracellular expression. However, in this case less cleaning and washing is needed compared with cytoplasmic products.

### Chemical synthesis of polypeptides.

Solid-phase method of the chemical synthesis of polypeptides comes to the fact that polypeptide end amino acids are bound with a carrier which is non-soluble in the given environment. While performing interaction between solid and liquid phases, a polypeptide chain is elongated up to the set length and separated from the carrier. The necessity or cleaning an intermediate product is excluded here, and this stipulates a sharp reduction of labour-intensiveness and duration of the technological process.
The synthesis of polypeptides in solid phase with further purification represents considerable difficulties within the present stage of biochemistry development, and the length of pure polypeptide chains which can be obtained does not exceed 30-50 amino-acid residues yet.

The main principles and peculiarities of the solid-phase synthesis of polypeptides.
In the solid-phase synthesis, the growing peptide chain is connected with a polymeric carrier by C-terminus, for example, with the use of Boc-amino acids and chloromethylated styrene copolymer and divinylbenzene. Primarily, a Boc-derivative reacts with chloromethylpolystyrene with the formation of ester groups. Then Boc-groups are selectively eliminated, and the formed polymeric aminocomponent is condensed with the second Boc-amino acid that results in peptide bond formation. Removal of protection groups and condensation with N-protected amino acids arc repeated up to the formation of an oligopeptide of the desired size. Hydrolysis of ester groups binding a peptide to a carrier is carried out after that, and then the peptide shall be separated and purified.

Indisputable advantage of the solid-phase synthesis is the possibility to perform operations in one reactor; this fact determiners its simplicity, efficiency and speed. Since the main stage of a chemical reaction takes place directly on a polymer, redundant reagents can be easily removed in washing. An important drawback of this method is that it is difficult to achieve 100% conversion and intermediate amino acids are being lost within expansion of a peptide chain, or defective peptides are being formed in which chain elongationhas stopped. Also, a product purification becomes more complicated after separation from a polymer. This creates considerable technological difficulties in the synthesis of polypeptides and enzymes. However, introduction of additional purification steps allows to produce physiologically active peptides rather successfully, as well as their analogs and active fragment, or low-molecular peptides by means of solid-phase method.

### Carriers and introduction of the functional groups.

The principle requirement to a carrier lies in the fact that it must not dissolve in solvents, possess chemical durability, be well permeable for reagents, be rather stable and be easily removed after any reaction. Styrene copolymer with divinylbenzene meets this requirement (ligation degree 1—2%, powder particles size - 200-400 mesh).

A wide range of methods is known for the introduction of the functional groups in order to form a covalent bond with carboxyl groups of amino acids. Among them, a method of chloromethylation of aromatic styrene polymer rings is known performed by means of morochlordimethyl ether under catalytic effect of SnCl₄.

Total volume of reagents, reaction time and temperature of the reaction shall be adjusted on such level to make Cl content per 1 g equal to 0.5-2.0 millimole.

More than 100 types of different carries are developed at present. Among them, oxymethylated polymer is widely recognized - in synthesis of oligopeptides, as well as oxymethylphenylacetamidomethylated polymer (shortly: oxymethyl-Pam) - for synthesis of long-chain peptides, and benzhydrolaminated polymer - for synthesis of peptides with amide group on C-terminus.

### Protective groups of amino acids.

In the starting period of polypeptide synthesis development by means of a solid-phase method, benzyloxycarbonyl groups only were used for protection of α-amino acids. However, due to the necessity to perform all operations in possibly more mild conditions these groups are being gradually substituted by Boc-groups. Boc-amino acids are also known which are received by means of (Boc)₂0 and Boc-ON being convenient for such method.

Moreover, 2-(*n*-xenyl)-2 propyloxycarbonyl, 9-fluorenylmethyloxycarbonyl and many other groups are used successfully for the protection of α-amino acids.

The functional groups of lateral (side) chains of amino acids must be protected. It is desirable for the protective groups of side chains to be stable in the process of peptide bonds formation and not damaging a peptide in its separation from a polymer. Thus, for example, if β-amino groups of lysine are protected by Z-groups, then in conditions corresponding to the removal of Boc-groups minor decomposition of the latter takes place followed by the formation of a by-product along with a target peptide. Therefore, the use of Z-groups must be limited by the synthesis of simple peptides only, the complex peptides shall be synthesized with the use of more stable 2-chlorobenzyloxycarbonyl groups (shortly: 2-ClZ) or analogs thereof.
BzI groups are rather efficient for protection of SH-groups. They retain stability in condition of Boc-groups removal. However, they are removed not completely even in the final phase of the usual processing (by means of hydrogen fluoride within 1 hour at 0°C); therefore, more severe conditions must be created (HF, 20°C, 20 min), or liquid ammonia must be used for processing. In case a peptide is damaged, then SH-groups must be protected by 4-metoxybenzyl groups (4-MeOBzl) which can be removed more easily by means of hydrogen fluoride processing.

### Introduction of C-terminal amino acids into a polymer.

The most popular is Merrifield method that involves binding of Boc-amino acids with chloromethylpolystyrene in methanol and ethyl acetate in the presence of NEt₃ under reflux during 25-26 hours. This method is rather simple and is used practically for all amino acids. It is necessary to take into account that it is unacceptable for Boc-His(Bzl),-OH and Boc-Met-OH due to the incidental alkylation of the side chains. When introducing C-terminal amino acids into a polymer by means of this method Boc-Asn- OH are formed Boc-Asp(OEt)-polymer and Boc—Asp - polymer with rather high yields. If DMF is used instead of ethanol, the reaction proceeds easily and smoothly at room temperature, and secondary processes do not almost take place (except for S-alkylation of methionine).

In etherification, side effect of chlormethyl groups with NEt₃ is possible which is finished by the formation of a quaternary ammonium salt. In order to prevent this, NEt is used in small amounts. 0.1-0.5 millimole of amino acid with Boc-groups is injected into a polymer comprising 0.5-2.0 millimole of chlorine. The substitution of NEt by a tetramethyl ammonium salt, potassium salt or cesium salt of Boc-amino acid, carrying out reaction in DMF allows to avoid formation of quaternary ammonium salts as by-products.

Quantitative assay is carried out in the following way: hydrolyzation by hydrogen fluoride or 12 M HCl mixture with propionic acid, or by 12 M HCl mixture with acetic acid and phenol (2:1:1) is curried out. In this case, carboxyl group is activated into DMF when a Boc-amino acid, including as well Boc—His(Bzl) -OH and Boc—Met—OH, is introduced into oxymethylated polymer. When adding dimethylaminopyridine (1 eq.), the process is going on quantitatively. In case non-reacted OH-groups remain in polymer, then in DCC using adverse reactions take place which need to be excluded by means of OH-groups blockage with acetyl and similar groups. The method based on DCC use allows to bind a Boc-amino acid with 4-oxymethyl-Pam-polymer).

One of the usual methods of the solid-phase synthesis lies in the fact that polymer is first of all bound with one amino acid obtaining in this case an initial platform for peptide synthesis. Also, there is a method in which acylpeptide is directly bound with a polymer. Reactive capacity of a bromomethylated polymer is higher than of a chloromethylated polymer in introduction of acylpeptides.

### Elongation of peptide chains.

Protective groups need to be removed before synthesis start. In order to remove protective Boc-groups of Boc-amino acids or peptides 1 M HCl mixture with acetic acid and 4 M HCl mixture with dioxane or a mixture of 25% TFA solution with CH₂Cl₂ are introduced into the reaction. After that, mixing in shaking is carried out. While performing the solid-phase synthesis of peptides containing tryptophan, there are difficulties connected with the fact that tryptophan oxidizes easily in acid medium. Addition of 2-mercaptoethanol and the process implementation in nitrogen atmosphere allow to prevent tryptophan decomposition to a large degree. In removal of Boc-groups by means of acids, benzylether bond of peptides' C-terminus with a polymer may be subject to hydrolysis, especially in case when C-terminus contains glycine. At the same time it is determine that ether bond is 100 times more stable in 4-oxymethyl-Pam-polymer than in polymethylene- or polyoxymethylenechloride. Peptides with Fmoc-groups are stable to acid effect in comparison with peptides protected by Boc and Bpoc groups; and the removal of the groups mentioned shall be carried out by means of the mixture of 50% piperidine-CH₂-Cl or mixture of 20% piperidine-DMF.

Different ways and methods are used for the consecutive lengthening of a peptide chain on a polymer. The use of MA, DCC and activated ethers are combined in various ways in these methods.

A preferable method is based on DCC use. However, if DCC is used in reactions with Boc-Asn (or Gln)-OH, dehydration of w-amide groups with their transformation into nitrile groups takes place. In order to avoid this, the corresponding activated ethers (for example, ONp) are used as a rule. At the same time, condensation method (protection of side groups) of asparagine and glutamin under DCC effect is used frequently.

While using Boc-amino acids the reaction capacity of which is known to be little, double repetition of the reaction is carried out usually. The use of 3 equivalent Boc-amino acids and DCC shall be optimal. In this case, reaction proceeds within 1-2 hours at 25°C. Addition of HONSu, as well as HOBt is rather efficient in ratio of polymer/solvent 1 g/10 ml. Condensation reactions are successfully carried out with the use of Boc-amino acids' anhydrides. They arc synthesized from an appropriate Boc-amino acid and DCC (2 and 1 equivalent respectively). In this case, non-soluble urea derivatives are not formed during the reaction and the process goes on at high speed. Reactions with activated ethers proceed more slowly than with DCC, however, addition of HOBt allows to accelerate the process and make it duration equal to 4-5 hours. What concerns stability and other parameters, rather satisfactory results are achieved in the use of *n*-nitrophenyl ethers with DMF as a solvent.

Upon completion of a peptide chain elongation it is necessary to separate a target peptide from a polymer, i.e. destroy an ether bond between them first of all. The ways of such splitting are determined by the nature of protective groups and the type of acid residues, as well as the purpose of the synthesized peptide.
Thus, for example, acidolysis with the use of hydrogen fluoride, HBr-AcOH and CF3₂S0₃H-TrA systems as well as other reagents shall be carried out in order to obtain a peptide with a free C-terminus. Ammonolysis and hydrazinolysis arc performed to receive amides and hydrazides respectively.
Hydrazides of protected peptides obtained in processing by hydrazine may be used as a material for condensation of fragments while performing liquid-phase synthesis. Splitting of ether bonds can be carried out also by interesterification and photolysis methods.

The most important issue of synthesis in general is the purity of a product. This relates considerably to the products produced by solid-phase processes. Almost in each case a good cleaning is achieved while combining gel chromatography and ion-exchange chromatography with HPLC method. Methods with the use of CCD, affinity chromatography and the method of impurities removal by enzymatic degradation are also efficient.

Consequently, both cloning in expression vectors and synthesis by means of a solid-phase method provide for the production of peptides similar to their natural analogs with regard to activity, and thus allow to use them in *in vitro, in vivo, ex vivo* investigations.

Thus, the peptides according to present invention may be synthesized with the use of known synthesis methods while possessing broader functional capabilities that is conditional on vivid immunosuppressive features in relation to broader range of specific links of immune system.

## Claims

1. Immunosuppressive peptide of the formula with variable positions **characterized in that** the formula with two (X) or three (Y) amino-acid replacements : 
D-loop C-loop N-loop

| | | * disulfide bond * | |

| | | | | | |

X1-L-D-X2-L-T-A-X3-Y1-G-G-Y2-C-Y3-X4-L-Y4-E-X5-C-C-X6-X7-Y5-N-Q-S-G-Y6-V

free
 X1={G,A}
X2-{L,M}
X3={E,A}
X4={F,A}
X5-{E,K}
X6-{F,Y}
X7={Y,W}
Y1={K,R,Q}
Y2={L,I,T}
Y3={L,l,T}
Y4={G,D,N}
Y5={V,L,T}
Y6={I,L,K}.

2. Immunosuppressive peptide according to Claim 1, charactezed in that for C and D type exogenous retroviruses (Human T-cell leukemia virus type I/II, Simian Mason-Pfizer virus, Simian retrovirus SRV-1/2, Squirrel monkey retrovirus) the formula is: 
D-loop C-loop N-loop

* disulfide bond *

G-L-D-L-L-X1-X2-E-Q-G-G-X3-C-X4-A-X5-Q-E-X6-C-C-F-X7-X8-N-X9-S-X10-X11-X12
 X1={F,T}
X2={W,A}
X3-{L,I}
X4={L,K}
X5={L,I}
X6={Q,K}
X7={L,Y}
X8={A,-}
X9={I,K}
X10={N,G}
X11={I,T,S}
X12={V,H}.

3. Immunosuppressive peptide according to Claim 1, **characterized in that** peptide library is: 
G-L-D-L-- L-X1-X2-E--Q--G-G-X3-C-X4-A--X5-Q--E-X6-C-C-F--X7-X8- N-X9-S-X10-X11-X12
X1-L-D-X2-L-T--A--X3-Y1-G-G-Y2-C-Y3-X4-L--Y4-E-X5-C-C-X6-X7-Y5-N-Q--S-G---Y6--V
### # ### # # ### # #,
 where # - designates coinciding amino-acid residues.
